**Europäisches Patentamt**

**European Patent Office**

**Office européen des brevets**

(19)

(11) Veröffentlichungsnummer: **0 023 580**
**B1**

(12) ## EUROPÄISCHE PATENTSCHRIFT

(45) Veröffentlichungstag der Patentschrift:
**06.02.85**

(51) Int. Cl.⁴: **A 61 M 5/14,** A 61 M 25/00,
A 61 M 25/02

(21) Anmeldenummer: **80103803.5**

(22) Anmeldetag: **04.07.80**

(54) **Katheteransatz.**

(30) Priorität: **24.07.79 DE 2929886**
**10.04.80 DE 3013871**

(43) Veröffentlichungstag der Anmeldung:
**11.02.81 Patentblatt 81/6**

(45) Bekanntmachung des Hinweises auf die Patenterteilung:
**06.02.85 Patentblatt 85/6**

(84) Benannte Vertragsstaaten:
**AT BE CH DE FR GB IT LI LU NL SE**

(56) Entgegenhaltungen:
**DE - A - 2 116 108**
**DE - A - 2 433 767**
**DE - A - 2 527 947**
**DE - A - 2 609 293**
**DE - A - 2 852 843**
**DE - C - 2 609 112**
**DE - U - 7 333 964**
**DE - U - 7 541 266**
**DE - U - 7 715 578**
**US - A - 4 015 600**

(73) Patentinhaber: **Intermedicat GmbH, Gerliswilstrasse 45,**
**CH-6020 Emmenbrücke (CH)**

(72) Erfinder: **Herlitze, Gerhard, Binsdörferstrasse 4,**
**D-3507 Baunatal/Guntershausen (DE)**

(74) Vertreter: **von Kreisler, Alek, Dipl.-Chem. et al,**
**Deichmannhaus am Hauptbahnhof, D-5000 Köln 1 (DE)**

## Beschreibung

Die Erfindung betrifft einen Katheteransatz zur Verwendung in Venen-Punktionsgeräten, bei dem ein Katheterschlauch oder eine Metallkanüle an einem hohlen Ansatzstück befestigt ist, das zwei miteinander verbundene, im wesentlichen ebene Platten trägt, die bei auf der Haut des Patienten befestigtem Ansatzstück nach zwei entgegengesetzten Seiten gerichtet auf der Haut aufliegen und die aus dieser Stellung hochschwenkbar sind.

Katheteransätze werden für Venen-Kurzkatheter oder Venen-Verweilkatheter mit flexiblem Katheterschlauch oder mit Metallkanüle verwendet. Bei Anbringung eines Katheterschlauches an dem Katheteransatz ist es zur Punktion eines Blutgefässes erforderlich, zusätzlich eine Metallkanüle einzusetzen, die durch den Katheteransatz und den Katheterschlauch hindurchgeschoben wird, wobei der Metallkanülenansatz eine Griffplatte aufweisen kann. Nach erfolgreicher Punktion wird die Metallkanüle aus dem Venen-Kurzkatheter oder Venen-Verweilkatheter zurückgezogen, und danach wird der Katheterschlauch in das Blutgefäss vorgeschoben. Der so gelegte Venen-Katheter wird nun auf der Punktionstelle fixiert. Zu diesem Zweck dienen von dem Ansatzstück nach entgegengesetzten Seiten gerichtete Fixierungsplatten, die nach der Punktion eines Blutgefässes auf der Hautoberfläche des Patienten liegen und auf dieser mittels Pflaster festgeklebt werden (DE-C3-2 609 112). Diese Fixierungsplatten sind über Stege miteinander und mit dem Ansatzstück fest und unbeweglich verbunden. Eine unbewegliche Anordnung der Fixierungsplatten ergibt sich auch durch deren Aufklemmen mit Klammern auf einen schlauch- oder metallkanülenseitigen Fortsatz des Katheteransatzes (DE-U-7 333 964). Ausserdem ist der eingangs erwähnte Katheteransatz mit in zwei zueinander senkrechte Stellungen beweglichen Platten bekannt. Dabei wird die Beweglichkeit durch flexible Flügel erreicht, die zum Punktieren um zur Längsachse des Katheteransatzes parallele Knicklinien gegeneinander nach oben geklappt werden und in dieser Stellung als Einführhilfe dienen (DE-A1-2 116 108 für Katheterschlauch, DE-A1-2 433 767 für Metallkanüle). Das Hochklappen der einzelnen Flügel ist umständlich. Ausserdem liegen in diesem Falle die hochgeklappten Flügel in Längsrichtung des Ansatzstückes aneinander an. Dies ist nachteilig, weil bei Ausübung der zum Punktieren notwendigen Vorschubkraft die Finger des Anwenders leicht von den Flügeln abrutschen können. Bei zusätzlicher Verwendung einer Metallkanüle mit Griffplatte ergibt sich, dass diese Griffplatte zu den hochgeklappten Flügeln des Katheteransatzes quer verläuft, so dass die hochgeklappten Flügel des Katheteransatzes nicht gleichzeitig mit der Griffplatte des Kanülenansatzes zwischen Daumen und Zeigefinger erfassbar sind, und aus diesem Grunde kann bei Verwendung dieses bekannten Katheteransatzes mit Katheterschlauch zur Punktion nur mit einer Metallkanüle ohne Griffplatte gearbeitet werden. Dies ist nachteilig, weil die Metallkanüle während der Punktion nicht festgehalten werden und sich in Längsrichtung verschieben kann. Es ist sicherer, Venen-Katheter zu benutzen, bei denen sowohl der Katheteransatz als auch die Metallkanüle mit Platten versehen sind, damit Daumen und Zeigefinger die Platten gleichzeitig erfassen können und während der Punktion der Katheteransatz gegen den Kanülenansatz gedrückt wird, so dass eine Längsverschiebung der Teile relativ zueinander verhindert wird. Dies ist bisher nur mit einer starr an dem Katheteransatz befestigten Griffplatte möglich, die nicht als Fixierungsplatte benutzbar ist. (DE-C3-2 609 112).

Der Erfindung liegt die Aufgabe zugrunde, einen Katheteransatz der eingangs erwähnten Art so auszubilden, dass die schwenkbaren Platten, die sowohl als Fixierungselemente als auch als Punktionshilfe dienen sollen, zu dem Katheteransatz so angeordnet sind, dass sie sich rutschsicher festhalten lassen und eine zuverlässige Handhabung während der Punktion ermöglichen.

Diese Aufgabe wird dadurch gelöst, dass die beiden Platten starr miteinander verbunden und über eine zu der Längsachse des Katheteransatzes quergerichtete Achse an dem Ansatzstück gelagert sind und dass ein Anschlag vorgesehen ist, der die Platten in hochgeschwenkter Stellung an dem Ansatzstück abstützt.

Die in dieser Weise beweglich an dem Ansatzstück des Katheteransatzes gelagerten Platten werden als Einheit zwangsläufig immer gemeinsam verschwenkt, wodurch sich eine Erleichterung der Handhabung ergibt. In der ersten Stellung liegen die beiden Platten flach auf der punktierten Hautoberfläche eines Patienten und dienen als Fixierungselemente zur Fixierung des Katheteransatzes auf der Haut mit Klebepflastern. In der um etwa 90° zu dieser ersten Stellung hochgeschwenkten zweiten Stellung verlaufen die beiden Platten als Einheit quer zur Längsachse des Katheteransatzes, so dass sie sich vom Anwender bei Ausübung der zur Punktion eines Blutgefässes erforderlichen Druckkraft rutschsicher und bequem erfassen und halten lassen. Der Anschlag bildet ein Widerlager für die Platten, das die beim Vorschieben der Anordnung durch den Zeigefinger eingeleitete Kraft aufnimmt und ein unbeabsichtigtes Umklappen der Platten nach vorne mit Sicherheit verhindert. Die Angriffsflächen von Daumen und Zeigefinger üben über einen Hebelarm zum Drehpunkt der Platten bei Krafteinleitung ein Drehmoment aus, das jedoch aufgrund der Abstützung der Platten an dem Ansatzstück durch ein Gegenmoment innerhalb der flächig aufsitzenden Finger ausreichend aufgehoben wird, so dass die Platten nicht nach vorne umklappen. Wird zur Punktion eine Metallkanüle mit Griffplatte eingesetzt, so liegen die beiden Platten des Katheteransatzes und die Griffplatte zueinander parallel und lassen sich zwischen Daumen und Zeigefinger einklemmen, wodurch beide Teile axial zusammengedrückt werden und der flexible Katheter während der Punktion festgehalten wird und sich relativ zur Metallkanüle nicht längsverschieben kann. Die Punktion des Blutgefässes wird mit Hilfe der in erfindungsgemässer Weise schwenkbar gelagerten Platten sowohl bei Verbindung des Katheteransatzes mit einer Metallkanüle als auch mit einem flexiblen Katheterschlauch mit innenliegender Metallkanüle sicherer.

Bei herabgeschwenkten Platten lässt sich der Katheteransatz in einer flachen Verpackung unterbringen und platzsparend aufbewahren.

Vorteilhafterweise besteht der Anschlag aus einem Bügel, der die beiden Platten miteinander verbindet und dessen der Achse zugewandtes Ende bei hochgeschwenkten Platten gegen das Ansatzstück anliegt. Die axiale Länge des Bügels ist so bemessen und er ist auf den Platten so angeordnet, dass die beiden Platten bei Anlage des Bügelendes gegen das Ansatzstück ihre um etwa 90° hochgeschwenkte Stellung einnehmen, in der sie als Punktionshilfe dienen. Gegebenenfalls kann der Bügel durch eine Anschlagnase verlängert sein, die mit einer geraden Auflagefläche auf dem Ansatzstück zusammenwirken kann, damit sich eine flächige Anlage beider Teile und nicht nur eine aus Stabilitätsgründen ungünstige Punktberührung mit dem runden Ansatzstück ergibt.

Der Bügel ist vorteilhafterweise konisch und im Querschnitt halbkreisförmig gewölbt gestaltet, wobei der Durchmesser seines der Achse zugewandten Endes grösser ist. Bei auf der Haut eines Patienten liegenden Platten liegt der Bügel auf einem axialen Fortsatz (Schutzkappensitz) des Ansatzstückes auf und das Ansatzstück steht schräg nach hinten hoch. Die beiden Platten verlaufen also in der erwähnten Position in bezug auf die Längsachse des Ansatzstückes von hinten unten schräg nach oben vorne. Vorteilhafterweise beträgt der Winkel zwischen der Längsachse des Ansatzstückes und den liegenden Platten 5° bis ca. 20°. Dies bedeutet, dass bei auf der Hautoberfläche festgeklebten Platten z. B. ein an dem Katheteransatz befestigter Katheterschlauch unter einem dem Punktionsort angepassten Neigungswinkel gegenüber der Auflagefläche schräg angestellt ist. Dadurch wird ein starkes Abknicken des flexiblen Katheterschlauches nach Einführung in die Vene vermieden. Durch Veränderung der Lage der Achse kann der Winkel variiert werden.

Bei einer Weiterbildung der Erfindung sind die Platten in beiden Stellungen mittels Arretierungsnocken am Ansatzstück sicherbar. Die Arretierungsnocken verhindern ein unbeabsichtigtes Verschwenken der Platten aus der einen in die andere Stellung.

Die Achse ist bei der ersten vorteilhaften Ausgestaltung der Erfindung als Drehachse ausgebildet und an dem in hochgeschwenkter Stellung unteren Rand der Platten an die Unterseite des Ansatzstückes tangential verlaufend angesetzt. Die Drehachse besteht aus seitlichen Lagerzapfen an dem Ansatzstück oder den beiden Platten, die in seitliche Lageröffnungen an den beiden Platten oder in dem Ansatzstück eingreifen. Die Lagerzapfen oder die Lageröffnungen jeder Platte sind vorteilhafterweise an zu den Platten im wesentlichen senkrechten Laschen ausgebildet.

Die Lageröffnungen in den Laschen oder in dem Ansatzstück können an einer Seite klauenartig offen ausgebildet und auf die Lagerzapfen an dem Ansatzstück oder an den Laschen elastisch einschnappend aufsteckbar sein. Hierdurch ergibt sich eine einfache Montage der Platten an dem Ansatzstück.

In zweckmässiger Ausgestaltung der Erfindung kann die Drehachse als einstückiges Teil ausgebildet sein, das in einer Lageröffnung des Ansatzstückes gelagert ist. Die Drehachse kann elastisch biegbar ausgebildet sein. Die Biegbarkeit der Drehachse kann sich entweder über ihre ganze Länge oder nur an ihren Enden auswirken. Durch diese konstruktive Auslegung der Drehachse wird erreicht, dass das Ansatzstück mehr oder weniger fest zwischen den Platten fixiert wird und damit Zug- und Druckbewegungen, die von einem an das Ansatzstück angeschlossenen Infusionsgerät auf den in ein Blutgefäss eingesetzten Katheter ausgeübt werden, ausgeglichen werden.

Ferner geht es bei der Erfindung darum, die beiden Platten an dem Ansatzstück so zu lagern, dass sie ihre beiden Funktionen zufriedenstellend erfüllen und zusätzlich eine preiswerte Herstellbarkeit des gesamten Katheteransatzes erzielt wird.

Zu diesem Zweck ist vorgesehen, dass die Achse eine Materialschwächungslinie ist, die einen Plattenkörper aus flexiblem Material in ein mit dem Ansatzstück verbundenes Trägerteil und die schwenkbaren Platten unterteilt.

Der Plattenkörper mit der bei seiner Herstellung anbringbaren Materialschwächungslinie bildet ein einfaches Teil, das sich billig herstellen lässt. Durch Knickung des Plattenkörpers längs der Materialschwächungslinie lassen sich die beiden Platten zu dem auf der Haut des Patienten liegenden, mit dem Ansatzstück verbundenen Trägerteil hochschwenken, so dass sie als Punktionshilfe benutzbar sind. In ihrer Grundstellung liegen die beiden Platten in der Ebene des Trägerteiles. Dies ist vorteilhaft, weil das Trägerteil die Platten in Richtung der Längsachse des Ansatzstückes verlängert, so dass sich bei Befestigung des Ansatzstückes mittels Klebepflastern auf der Haut eines Patienten eine vergrösserte Auflage- und Befestigungsfläche ergibt. Dadurch wird die Fixierung eines Verweilkatheters erleichtert und verbessert.

Vorteilhafterweise ist vorgesehen, dass der Plattenkörper eben ist und dass in Draufsicht die Platten um das Trägerteil jeweils etwa rechteckige Form aufweisen. Alternativ können in Draufsicht das Trägerteil dreieckig oder trapezförmig und die Platten etwa rechteckig sein. In Längsrichtung des Ansatzstückes ist das Trägerteil vorteilhaft kürzer als die Platten. Dies ergibt sich durch entsprechende Anordnung der Materialschwächungslinie auf dem Plattenkörper und hat den Zweck, eine handliche Höhe der hochgeschwenkten Platten zu erzielen, ohne dass ein klobiger Plattenkörper benötigt wird. Die Haltefunktion eines schmalen Trägerteiles reicht zur sicheren Verbindung der Platten mit dem Ansatzstück aus.

Das Trägerteil kann mit dem Ansatzstück aus Kunststoff einstückig hergestellt und an der Unterseite des Ansatzstückes zu diesem tangential verlaufend angeordnet sein. Die Ecken des Plattenkörpers sind vorteilhafterweise abgerundet. Beide Platten und/oder das Trägerteil können wenigstens auf einer Fläche Rillenprofilierungen aufweisen, die die Flexibilität erhöhen, so dass der Plattenkörper sich dem Verlauf der Unterlage, auf der er befestigt ist, geschmeidig anpassen kann. Ausserdem wird hierdurch die Griffigkeit verbessert.

Da die beiden Platten relativ zum Trägerteil hochgeklappt werden, befindet sich die Materialschwächungslinie zweckmässig auf der Unterseite des Plattenkörpers. Sie kann durchgehend von einem zum anderen Ende jeder Platte verlaufen. In zweckmässiger Ausgestaltung kann die Materialschwächungslinie kürzer sein als jede Platte. Praktisch ist dann zwischen dem Trägerteil und der Platte nur noch ein schmaler Riegel der Materialschwächungslinie vorhanden, und die Schwenkbarkeit der Platten wird erleichtert.

Die Materialschwächungslinie kann als gerade Rille mit gleichmässiger Tiefe ausgebildet sein oder sie kann aus aneinandergereihten Vertiefungen oder Durchbrüchen bestehen. In jedem Falle ist lediglich wichtig, dass sie die Schwenkbarkeit der beiden Platten zu dem Trägerteil ermöglicht und sich auf einfache Weise an dem Plattenkörper anbringen lässt. Der Plattenkörper kann über seine ganze Erstreckung gleiche Dicke haben.

In der Zeichnung sind Ausführungsbeispiele der Erfindung schematisch dargestellt.

Es zeigt:

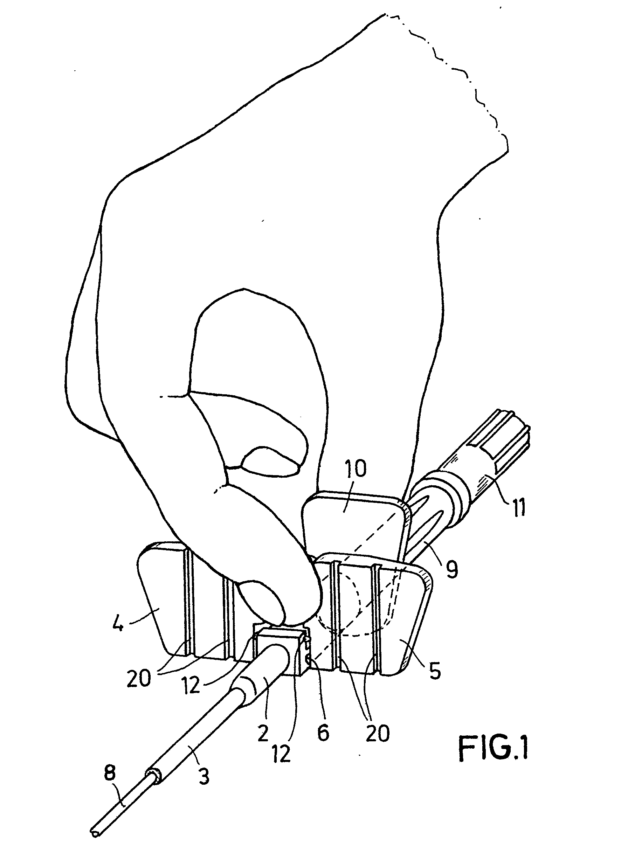

Fig. 1 eine schaubildliche Ansicht eines Katheteransatzes mit um eine Drehachse hochgeschwenkten Platten und in den Katheteransatz eingeschobener Metallkanüle während der Punktion eines Blutgefässes,

Fig. 2 eine schaubildliche Ansicht eines Katheteransatzes mit in die Punktionsebene herabgeschwenkten Platten, die auf der Haut des Patienten festgeklebt sind,

Fig. 3 bis 5 Seitenansicht, Rückansicht und Draufsicht eines in ein Blutgefäss eingeführten auf der Haut des Patienten festgelegten Katheteransatzes,

Fig. 6 und 7 Querschnitt ähnlich Fig. 12 und 18 zur Darstellung abgewandelter Ausführungsformen von Drehachsen eines Plattenpaares,

Fig. 8 eine Draufsicht eines Plattenpaares,

Fig. 9 eine Ansicht des Plattenpaares nach Fig. 8 in Richtung des Pfeiles A gesehen,

Fig. 10 eine Seitenansicht einer Platte des Plattenpaares nach Fig. 8 und 9,

Fig. 11 eine Draufsicht eines mit dem Plattenpaar nach Fig. 8 bis 10 zu bestückenden Katheteransatzes,

Fig. 12 einen Querschnitt des Ansatzstückes längs einer die Arretierungsnocken und Lagerzapfen durchquerenden Linie,

Fig. 13 einen Querschnitt des Ansatzstückes längs der Linie XIII–XIII in Fig. 11,

Fig. 14 eine Draufsicht einer anderen Ausführungsform eines Plattenpaares,

Fig. 15 eine Ansicht des Plattenpaares nach Fig. 14 in Richtung des Pfeiles B gesehen,

Fig. 16 eine Seitenansicht einer Platte des Plattenpaares nach Fig. 14 und 15,

Fig. 17 eine Draufsicht eines mit dem Plattenpaar nach Fig. 14–16 zu bestückenden Katheteransatzes,

Fig. 18 einen Querschnitt des Katheteransatzes nach Fig. 17 längs einer die Arretierungsnocken und Lageröffnungen durchquerenden Linie, und

Fig. 19 einen Querschnitt längs der Linie XIX—XIX in Fig. 17,

Fig. 20 eine schaubildliche Ansicht eines Katheteransatzes mit um eine Materialschwächungslinie hochgeschwenkten Platten und in den Kathetereinsatz eingeschobener Metallkanüle während der Punktion eines Blutgefässes,

Fig. 21 eine Seitenansicht der Darstellung nach Fig. 20,

Fig. 22 eine schaubildliche Ansicht des Katheteransatzes ohne Metallkanüle mit in Punktionsebene liegenden Platten, die auf der Haut eines Patienten festgeklebt sind,

Fig. 23 eine Seitenansicht der Anordnung nach Fig. 22, und

Fig. 24 eine Rückansicht der Anordnung nach den Fig. 22 und 23.

Der Katheteransatz zur Verwendung in Venen-Punktionsgeräten besteht im wesentlichen aus einem Ansatzstück 1 für den Anschluss von Übertragungsgeräten, z. B. einem Infusionsgerät o. dgl. und einem über einen Schutzkappensitz 2 fest mit dem Ansatzstück 1 verbundenen flexiblen Kunststoffschlauch 3. An dem Ansatzstück 1 sind zwei Platten 4 und 5 um eine zur Längsachse des Ansatzstückes 1 quer gerichtete Drehachse 6 drehbar gelagert. Die beiden Platten 4 und 5 sind im wesentlichen eben gestaltet und mittels eines etwa halbkreisförmig gewölbten Bügels 7 starr miteinander verbunden. Der Katheteransatz und das Plattenpaar sind aus physiologisch gut verträglichem Kunststoff hergestellt.

In dem Katheteransatz steckt eine Metallkanüle 8, die von einem Ansatz 9 getragen wird, an dem eine quer gerichtete Griffplatte 10 fest und unbeweglich angeformt ist. Auf das Ende des Kanülenansatzes 9 ist eine Verschlusskappe 11 aufgesteckt.

Zur Arretierung des Plattenpaares 4, 5 in der um 90° zur Punktionsebene hochgeschwenkten Stellung dienen zwei Arretierungsnocken 12, die an dem Ansatzstück 1 angeformt sind und hinter denen die beiden Platten 4 und 5 in der senkrechten Stellung einrasten. Als hinterer Anschlag dient entweder der der Drehachse 6 zugewandte Rand des Bügels 7 oder eine an diesem Rand vorgesehene Anschlagnase 13, die gegen eine ebene Auflagefläche 14 auf dem Katheteransatz 1 zur Anlage kommt.

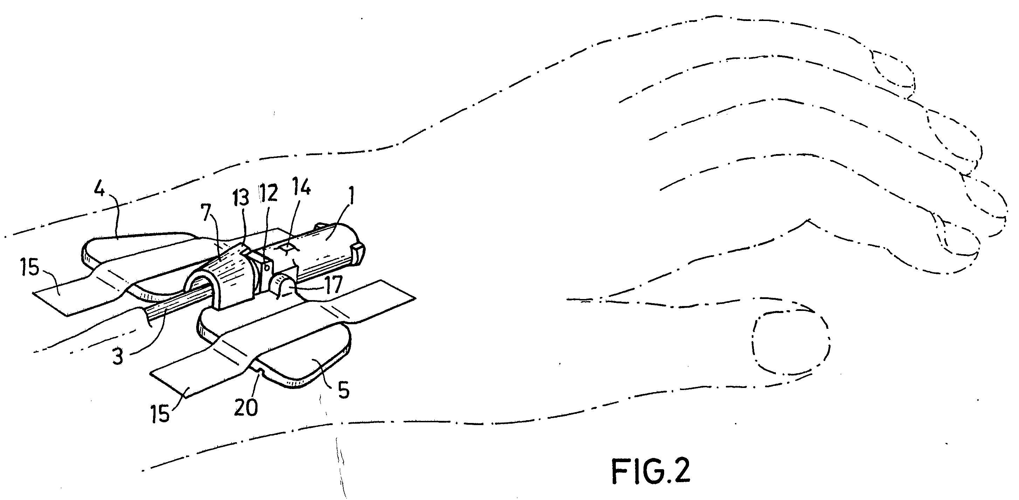

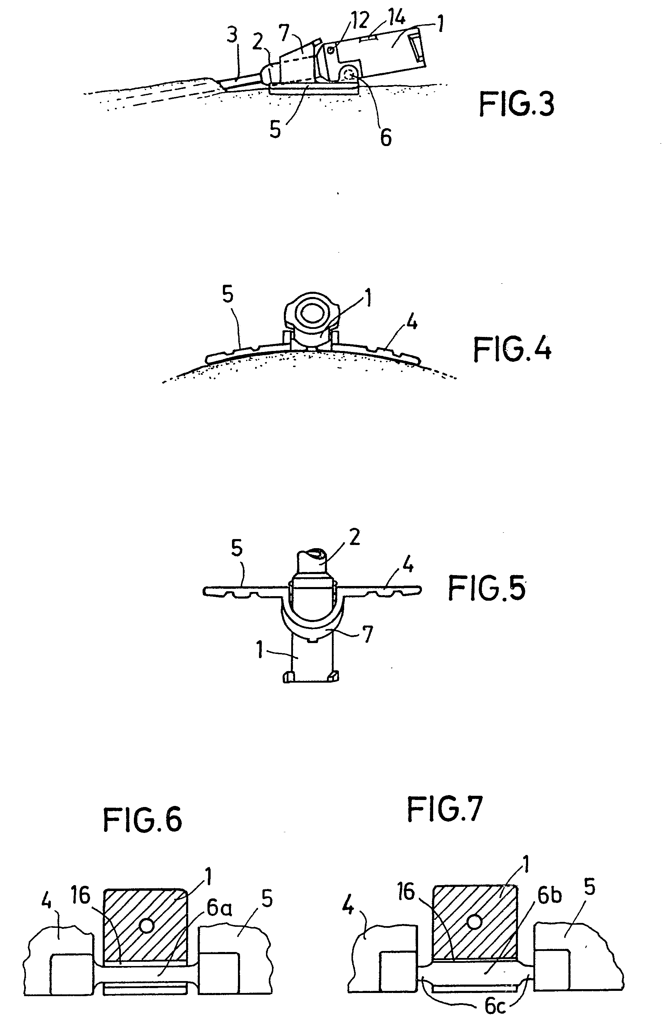

Zur Punktion eines Blutgefässes werden die Platten 10 und 4, 5 zwischen Daumen und Zeigefinger erfasst und der Katheteransatz mit eingesetzter Metallkanüle werden sicher gehalten in eine Vene eingeführt. Nach beendeter Punktion wird die Metallkanüle 8, 9, 10 aus dem Katheteransatz axial herausgezogen, und es wird der Schlauch 3 in die Vene eingeschoben. Die beiden Platten 4 und 5 werden in die Punktionsebene geschwenkt (Fig. 2), so dass sie flach auf der Haut eines Pateinten aufliegen und mittels Klebepflastern 15 auf der Haut befestigt werden können. In herabgeklapptem Zustand stehen der Schlauch 3 und das Ansatzstück 1 unter einem Anstellwinkel zur Oberfläche der Punktionsstelle (Fig. 3, 4 und 5), damit ein starkes Abknicken des Schlauches 3 vermieden wird. In Fig. 4, die eine Rückansicht des Katheteransatzes nach Fig. 3 darstellt, ist der zum Anschluss des Infusionsgerätes erforderliche Abstand des Katheteransatzes zur Hautoberfläche gut sichtbar. Der Anstellwinkel ist einmal von der Anordnung der Drehachse 6 in bezug auf das Ansatzstück 1 abhängig und durch Veränderung der Lage der Drehachse lässt er sich verändern. Zum anderen ist für den Anstellwinkel die Form des Bügels 7 wichtig, der zweckmässig als längsgeschnittener Kegelstumpf gestaltet ist, so dass sein der Drehachse 6 zugewandtes Ende grösseren Durchmesser hat als das gegenüberliegende Ende. Bei Anlage des Bügels 7 gegen den Schutzkappensitz 2 verlaufen daher beide Platten in bezug auf die Längsachse des Ansatzstückes von hinten unten nach vorne oben schräg angestellt bzw. bei waagerecht liegenden Platten erhält das Ansatzstück 1 mit dem flexiblen Schlauch 3 eine Abwärtsneigung in Richtung der Schlauchspitze.

Fig. 6 und 7 zeigen zwei mögliche Ausbildungen der Drehachse 6. Im Falle der Fig. 6 ist eine Drehachse 6a als einstückiges Teil ausgebildet, das in einer durchgehenden Lageröffnung 16 in dem Ansatzstück 1 drehbar gelagert und endseitig mit den Platten 4 und 5 fest verbunden ist. Die Drehachse hat im Bereich der Lageröffnung 16 in bezug auf diese einen verhältnismässig kleinen Durchmesser, so dass sie elastisch biegbar ist und Zug- und Druckbewegungen durch das angekoppelte Infusionsgerät ausgleicht. Bei dem Beispiel der Fig. 7 ist eine durchgehende Drehachse 6b drehbar in die Lageröffnung 16 des Ansatzstückes 1 eingepasst. Zur Erzielung elastischer Biegbarkeit im Bereich der Drehachse haben ihre mit den Platten 4, 5 verbundenen Enden 6c verringerten Durchmesser.

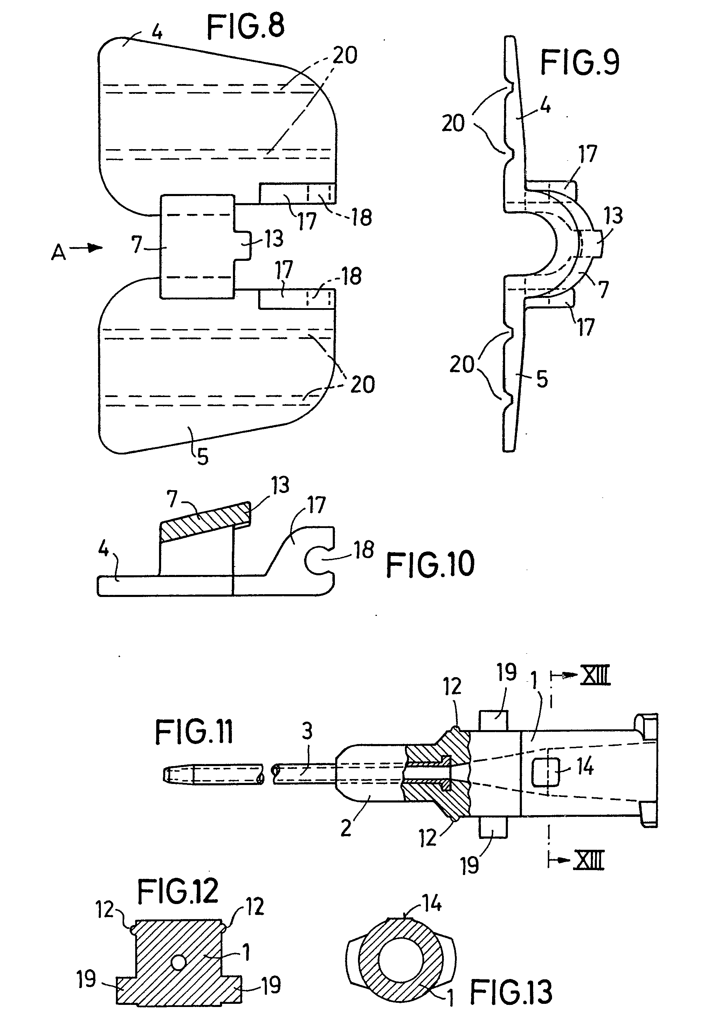

Andere Ausbildungen der Drehachse 6 sind in den Fig. 8 bis 13 und 14 bis 19 veranschaulicht.

Die in Fig. 8 in Draufsicht gezeigten Platten 4 und 5 haben Flügelform und sind mittels des angespritzten Bügels 7 starr miteinander verbunden. An dem inneren Rand jeder Platte 4, 5 ist an einem Ende jeweils eine senkrecht stehende Lasche 17 angeformt, in der eine Lageröffnung 18 ausgebildet ist. Die Lageröffnung 18 ist an einer Seite klauenartig offen. Den Lageröffnungen 18 angepasste kurze Lagerzapfen 19 befinden sich an dem Ansatzstück 1 und sind an dieses so angesetzt, dass sich ein tangentialer Verlauf der Drehachse in bezug auf die Unterseite des Ansatzstückes 1 ergibt (Fig. 12). Zur Verbindung der Platten 4 und 5 mit dem Ansatzstück 1 werden einfach die Lagerzapfen 19 in die offenen Lageröffnungen 18 in den Laschen 17 hineingedrückt und schnappen in diese ein. Die beiden Platten 4 und 5 sind sodann um 90° in senkrechte oder waagerechte Position zu dem Ansatzstück 1 schwenkbar. Zu ihrer Arretierung in senkrechter Position dienen zwei quer gerichtete Arretierungsnocken 12. Ein Ansatz 13 des Bügels 7 liegt in senkrechter Stellung der beiden Platten gegen eine ebene Auflagefläche 14 verschiebungssicher an. Auf der Unterseite jeder Platte 4 bzw. 5 sind Längsrillen 20 ausgebildet, die die Flexibilität und Grifffreundlichkeit der Platten 4 und 5 verbessern.

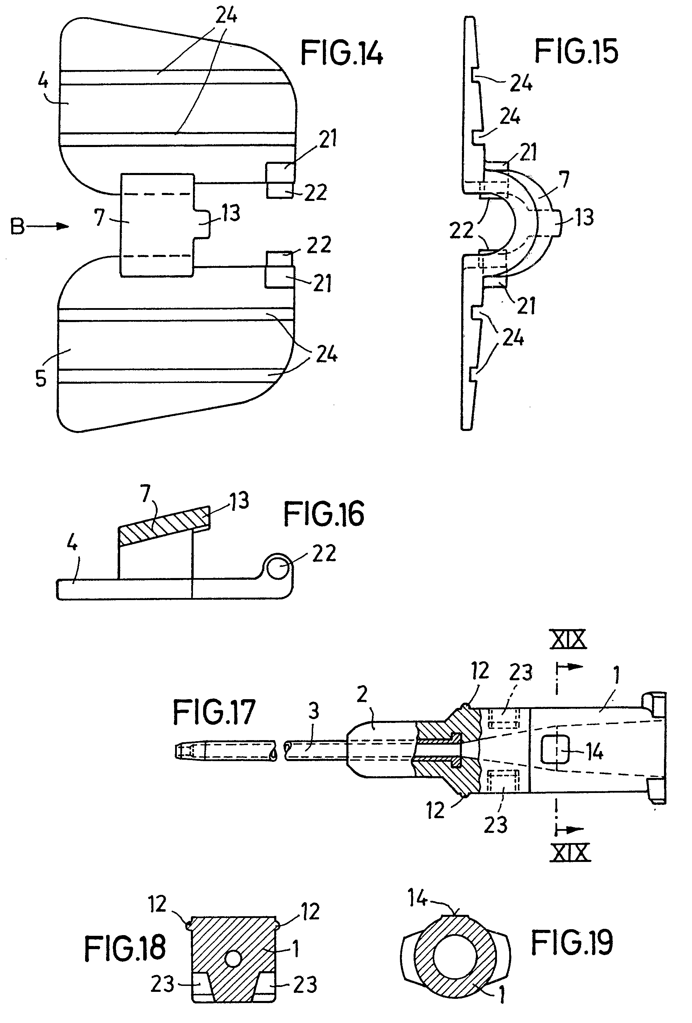

Gemäss Fig. 14 sind an stehenden Laschen 21 der beiden Platten 4 und 5 gegeneinander gerichtete Lagerzapfen 22 ausgebildet. Diese Lagerzapfen werden in nach unten klauenartig offene Lageröffnungen 23 im Ansatzstück 1 eingeführt. Beim Hineindrücken der Lagerzapfen 22 in die Lageröffnungen 23 spreizen sich die Klauen elastisch auseinander, so dass die Lagerzapfen 22 in die Lageröffnungen einschnappen können und drehbar in diesen gehalten sind. Bei diesem Beispiel sind Längsrillen 24 auf der Oberseite der Platten 4 und 5 ausgebildet. Zusätzlich oder alternativ können Querrillen vorgesehen sein. Ausserdem ist die Anordnung der Rillen auf der Ober- oder Unterseite der Platten unabhängig von der Ausbildung der Drehachse beliebig möglich.

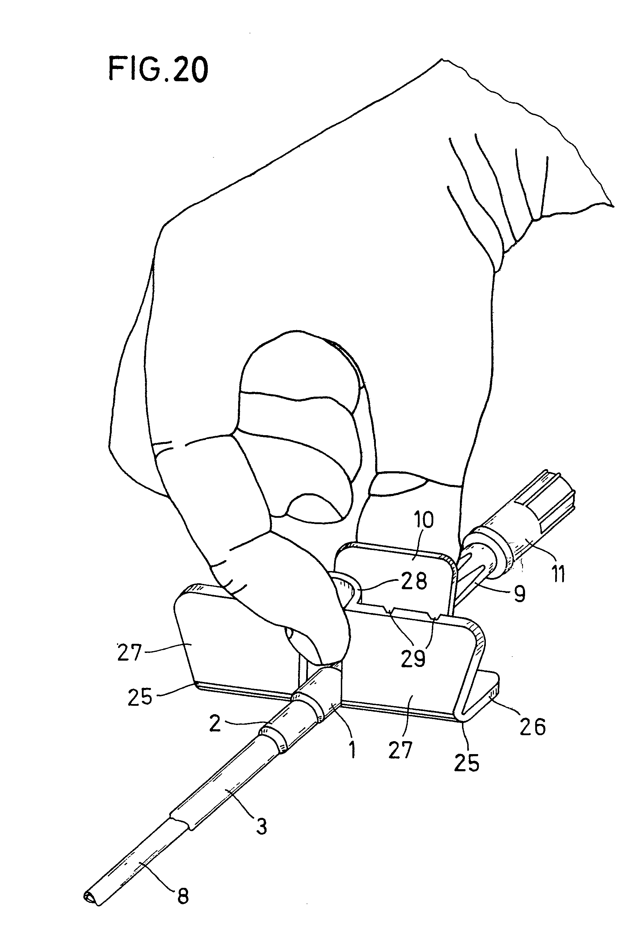

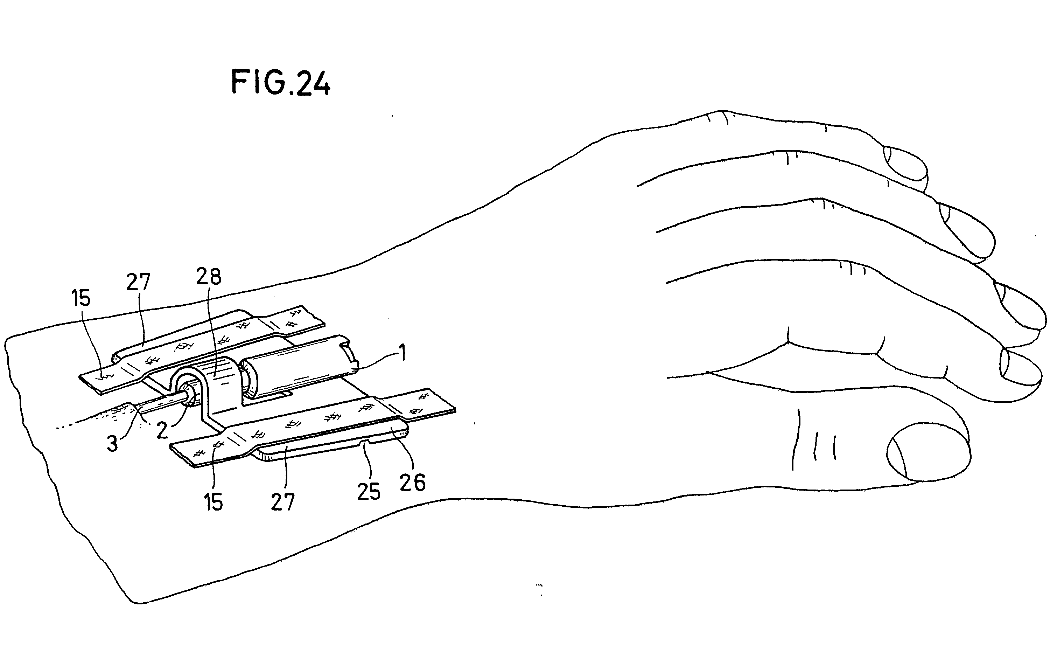

Der Katheteransatz gemäss Fig. 20 bis 24 besteht im wesentlichen ebenfalls aus einem Ansatzstück 1 und einem über einen Schutzkappensitz 2 fest mit dem Ansatzstück 1 verbundenen flexiblen Kunststoffschlauch 3. In dem Katheteransatz steckt eine Metallkanüle 8, die von einem Kanülenansatz 9 getragen wird, an dem eine nach oben gerichtete Griffplatte 10 fest und unbeweglich angeformt ist. Auf das Ende des Kanülenansatzes 9 ist während der Punktion eine Verschlusskappe 11 aufgesteckt.

Ein im wesentlichen rechteckiger Plattenkörper aus flexiblem Material ist mittels einer zur Längsachse des Ansatzstückes 1 quer gerichteten Materialschwächungslinie 25 in einem mit dem Ansatzstück 1 symmetrisch verbundenen Trägerteil 26 und ein um die Materialschwächungslinie 25 zu dem Trägerteil 26 schwenkbares Paar von Platten 27 mit Längsrillen 29 unterteilt.

Die beiden Platten 27 sind mittels eines nach oben gerichteten etwa halbkreisförmigen Bügels 28 starr miteinander verbunden. Der Bügel 28 beginnt im wesentlichen am vorderen Ende der Platten 27 und endet mit Abstand vor der Materialschwächungslinie 25. Auf diese Weise behindert der Bügel 28 das Hochschwenken der Platten 27 nicht, und sein hinterer Rand ist gemeinsam mit dem Ansatzstück 1 als Anschlag zur Fixierung der senkrechten Stellung der Platten 27 wirksam. Auf diese Weise bilden die Platten 27 in hochgeschwenktem Zustand eine starre Einheit mit dem Ansatzstück 1, die sich funktionssicher und einfach handhaben lässt.

Vorteilhaft ist der Trägerteil 26 mit dem Ansatzstück 1 aus Kunststoff einstückig ausgebildet. Bei der Herstellung werden die Materialschwächungslinie 25 und der Bügel 28 mit angeformt. Die Materialschwächungslinie 25 bildet ein Scharnier, das die Platten 27 mit dem Trägerteil 26 gelenkig verbindet. Die Materialschwächungslinie 25 ist bei dem gezeichneten Beispiel als gerade Rille gleichmässiger Tiefe mit trapezförmigem Querschnitt ausgebildet, die am Ansatzstück 1 über die Querbreite des Bügels 28 unterbrochen ist. Der Plattenkörper ist durch die Materialschwächungslinie 25 so aufgeteilt, dass der Trägerteil 26 etwa 1/3 und die Platten 27 etwa 2/3 der Breite des Plattenkörpers einnehmen. Der schmale Trägerteil 26 genügt zur Halterung der beiden Platten 27, und diese erhalten durch die erwähnte Aufteilung eine für die Handhabung bei der Punktion günstige Länge.

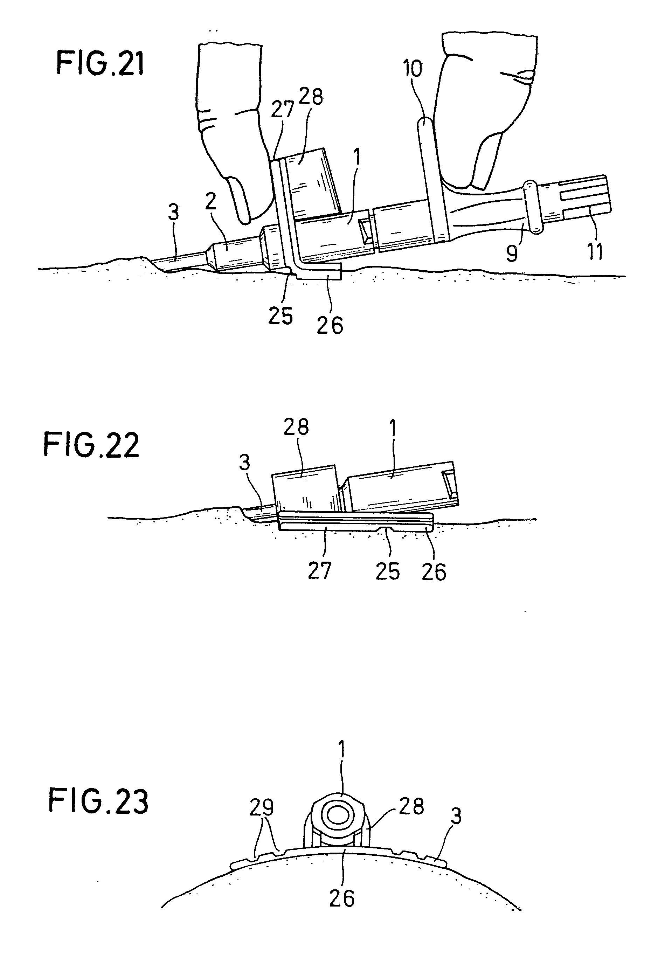

Gemäss Fig. 22 steht der ganze ebene Plattenkörper des Trägerteiles 26 und der beiden Platten 27 zur Fixierung des Katheteransatzes und des mit ihm verbundenen in ein Blutgefäss eingeführten Katheterschlauches 3 zur Verfügung. Über jeden der beiden in Punktionsebene liegenden Flügel des Plattenkörpers wird ein Klebepflaster 15 gelegt, das auf der Haut des Patienten befestigt wird. Es ergibt sich eine verschiebungssichere Festlegung des Verweilkatheters.

**Patentansprüche**

1. Katheteransatz zur Verwendung in Venen-Punktionsgeräten, bei dem ein Katheterschlauch (3) oder eine Metallkanüle an einem hohlen Ansatzstück (1) befestigt ist, das zwei miteinander verbundene, im wesentlichen ebene Platten (4, 5; 27) trägt, die bei auf der Haut des Patienten befestigtem Ansatzstück (1) nach zwei entgegengesetzten Seiten gerichtet auf der Haut aufliegen und die aus dieser Stellung hochschwenkbar sind, dadurch gekennzeichnet, dass die beiden Platten (4, 5; 27) starr miteinander verbunden und über eine zu der Längsachse des Katheteransatzes quergerichtete Achse (6, 25) an dem Ansatzstück (1) gelagert sind und dass ein Anschlag vorgesehen ist, der die Platten (4, 5; 27) in hochgeschwenkter Stellung an dem Ansatzstück (1) abstützt.

2. Katheteransatz nach Patentanspruch 1, dadurch gekennzeichnet, dass der Anschlag aus einem Bügel (7; 28) besteht, der die beiden Platten (4, 5; 27) miteinander verbindet und dessen der Achse (6; 25) zugewandtes Ende bei hochgeschwenkten Platten (4, 5; 27) gegen das Ansatzstück (1) anliegt.

3. Katheteransatz nach Patentanspruch 2, dadurch gekennzeichnet, dass der Bügel (7; 28) konisch und im Querschnitt halbkreisförmig gewölbt gestaltet ist, und dass der Durchmesser seines der Achse (6; 35) zugewandten Endes grösser ist.

4. Katheteransatz nach Patentanspruch 2 oder 3, dadurch gekennzeichnet, dass der Bügel (7, 28) bei auf der Haut des Patienten liegenden Platten (4, 5; 27) auf einem axialen Fortsatz (Schutzkappensitz 2) des Ansatzstückes (1) aufliegt und das Ansatzstück (1) schräg nach hinten hochsteht.

5. Katheteransatz nach Patentanspruch 4, dadurch gekennzeichnet, dass der Winkel zwischen der Längsachse des Ansatzstückes (1) und den liegenden Platten (4, 5; 27) 5° bis ca. 20° beträgt.

6. Katheteransatz nach einem der Patentansprüche 1 bis 5, dadurch gekennzeichnet, dass die Platten (4, 5) in beiden Stellungen mittels Arretierungsnocken (12) am Ansatzstück (1) sicherbar sind.

7. Katheteransatz nach einem der Patentansprüche 1 bis 6, dadurch gekennzeichnet, dass die Achse als Drehachse (6) ausgebildet und an dem in hochgeschwenkter Stellung unteren Rand der Platten (4, 5) an die Unterseite des Ansatzstückes (1) tangential verlaufend angesetzt ist.

8. Katheteransatz nach Patentanspruch 7, dadurch gekennzeichnet, dass die Drehachse (6) aus seitlichen Lagerzapfen (19; 22) an dem Ansatzstück (1) oder den beiden Platten (4, 5) besteht, die in seitliche Lageröffnungen (18, 23) an den beiden Platten (4, 5) oder in dem Ansatzstück (1) eingreifen.

9. Katheteransatz nach Patentanspruch 8, dadurch gekennzeichnet, dass die Lagerzapfen (19; 22) oder die Lageröffnungen (18; 23) jeder Platte (4, 5) an zu den Platten (4, 5) im wesentlichen senkrechten Laschen (17; 21) ausgebildet sind.

10. Katheteransatz nach Patentanspruch 8 oder 9, dadurch gekennzeichnet, dass die Lageröffnungen (18; 23) in den Laschen (17) oder in dem Ansatzstück (1) an einer Seite klauenartig offen ausgebildet und auf die Lagerzapfen (19; 22) an dem Ansatzstück (1) oder an den Laschen (21) elastisch einschnappend aufsteckbar sind.

11. Katheteransatz nach Patentanspruch 7, dadurch gekennzeichnet, dass die Drehachse (6) als einstückiges Teil (6a; 6b) ausgebildet ist, das in einer Lageröffnung (16) des Ansatzstückes (1) gelagert ist.

12. Katheteransatz nach Patentanspruch 11, dadurch gekennzeichnet, dass die Drehachse (6a; 6b) elastisch biegbar ausgebildet ist.

13. Katheteransatz nach Patentanspruch 1, dadurch gekennzeichnet, dass die Achse eine Materialschwächungslinie (25) ist, die einen Plattenkörper aus flexiblem Material in ein mit dem Ansatzstück (1) verbundenes Trägerteil (26) und die schwenkbaren Platten (27) unterteilt.

14. Katheteransatz nach Patentanspruch 13, dadurch gekennzeichnet, dass der Plattenkörper eben ist und dass in Draufsicht die Platten (27) und das Trägerteil (26) jeweils etwa rechteckige Form aufweisen.

15. Katheteransatz nach Patentanspruch 13, dadurch gekennzeichnet, dass der Plattenkörper eben ist und dass in Draufsicht das Trägerteil dreieckig oder trapezförmig und die Platten etwa rechteckig sind.

16. Katheteransatz nach Patentanspruch 13, dadurch gekennzeichnet, dass das Trägerteil (26) in Längsrichtung des Ansatzstückes (1) kürzer als die Platten (27) ist.

17. Katheteransatz nach Patentanspruch 13, dadurch gekennzeichnet, dass die Materialschwächungslinie (25) sich auf der Unterseite des Plattenkörpers befindet.

18. Katheteransatz nach Patentanspruch 17, dadurch gekennzeichnet, dass die Materialschwächungslinie (25) durchgehend von einem zum anderen Ende jeder Platte (27) verläuft.

19. Katheteransatz nach Patentanspruch 17, dadurch gekennzeichnet, dass die Materialschwächungslinie kürzer als jede Platte (27) ist.

20. Katheteransatz nach Patentanspruch 18 oder 19, dadurch gekennzeichnet, dass die Materialschwächungslinie (25) als gerade Rille mit gleichmässiger Tiefe ausgebildet ist.

21. Katheteransatz nach Patentanspruch 18 oder 19, dadurch gekennzeichnet, dass die Materialschwächungslinie aus aneinandergereihten Vertiefungen oder Durchbrüchen besteht.

22. Katheteransatz nach einem der Patentansprüche 13 bis 21, dadurch gekennzeichnet, dass das Trägerteil (26) mit dem Ansatzstück (1) aus Kunststoff einstückig hergestellt und an der Unterseite des Ansatzstückes (1) zu diesem tangential verlaufend angeordnet ist.

23. Katheteransatz nach einem der Patentansprüche 13 bis 22, dadurch gekennzeichnet, dass die Ecken des Plattenkörpers abgerundet sind, und dass die beiden Platten (4, 5, 27) und/oder das Trägerteil (26) wenigstens auf einer Fläche Rillenprofilierungen (20; 24; 29) aufweist.

**Claims**

1. Catheter attachment for use in venepuncture instruments comprising a catheter tube (3) or a metal cannula fixed to a hollow attachment member (1) bearing two interconnected substantially flat plates (4, 5; 27) which, when the attachment member (1) is fixed on the patient's skin rest on two oppositely directed sides on the skin and which may be tilted up from said position, characterized in that the two plates (4, 5; 27) are rigidly connected to each other and are supported at the attachment member (1) via an axis (6; 25) transversely directed to the longitudinal axis of the catheter attachment, and in that a stop is provided which supports the plates (4, 5; 27) in tilted up position at the attachment member (1).

2. Catheter attachment according to claim 1, characterized in that the stop comprises a shackle (7; 28) interconnecting the two plates (4, 5; 28) and whose end facing the axis (6; 25) rests against the attachment member (1) when the plates (4, 5; 27) are tilted up.

3. Catheter attachment according to claim 2, characterized in that the shackle (7; 28) is shaped conically and that it is curved in a semi-circular configuration in cross section, and in that the diameter of its end facing the axis (6; 35) is greater.

4. Catheter attachment according to claims 2 or 3, characterized in that the shackle (7, 28) rests on an axial projection (protective cap seat 2) of the attachment member (1), when the plates (4, 5; 27) lie on the patient's skin and the attachment member (1) is inclined upwardly to the rear.

5. Catheter attachment according to claim 4, characterized in that the angle between the longitudinal axis of the attachment member (1) and the lying plates (4, 5; 27) is from 5° to about 20°.

6. Catheter attachment according to one of claims 1 to 5, characterized in that the plates (4, 5) can be secured in both positions by means of locking cams (12) at the attachment member (1).

7. Catheter attachment according to one of claims 1 to 6, characterized in that the axis is designed as an axis of rotation (6) which, in tilted up position of the plates (4, 5) is mounted at their lower edge to extend tangentially to the underside of the attachment member (1).

8. Catheter attachment according to claim 7, characterized in that the axis of rotation (6) comprises lateral bearing journals (19; 22) on the attachment member (1) or the two plates (4, 5) which engage lateral bearing openings (18, 23) at both plates (4, 5) or in the attachment member (1).

9. Catheter attachment according to claim 8, characterized in that the bearing journals (19; 22) or the bearing openings (18; 23) of each plate (4, 5) are formed on brackets (17; 21) substantially perpendicular to the plates (4, 5).

10. Catheter attachment according to claims 8 or 9, characterized in that the bearing openings (18; 23) in the brackets (17) or in the attachment member (1) are open in a claw-type manner on one side and can be placed on the bearing journals (19; 22) at the attachment member (1) or on the brackets (21) in a resiliently catching manner.

11. Catheter attachment according to claim 7, characterized in that the axis of rotation (6) is an integral part (6a; 6b) which is mounted in a bearing opening (16) of the attachment member (1).

12. Catheter attachment according to claim 11, characterized in that the axis of rotation (6a; 6b) is resiliently bendable.

13. Catheter attachment according to claim 1, characterized in that the axis is a line of weakened material (25) which divides a plate body of flexible material into a carrier portion (26) connected to the attachment member (1) and the tiltable plates (27).

14. Catheter attachment according to claim 13, characterized in that the plate body is flat and that, seen in plan view, the shape of the plates (27) and of the carrier portion (26) is approximately rectangular each.

15. Catheter attachment according to claim 13, characterized in that the plate body is flat and that, seen in plan view, the carrier portion is triangular or trapezoidal and the plates are approximately rectangular.

16. Catheter attachment according to claim 13, characterized in that in longitudinal direction of the attachment member (1), the carrier portion (26) is shorter than the plates (27).

17. Catheter attachment according to claim 13, characterized in that the line of weakened material (25) is located at the underside of the plate body.

18. Catheter attachment according to claim 17, characterized in that the line of weakened material (25) runs continuously from one end to the other of each plate (27).

19. Catheter attachment according to claim 17, characterized in that the line of weakened material is shorter than each plate (27).

20. Catheter attachment according to claim 18 or 19, characterized in that the line of weakened material (25) is designed as a straight groove of uniform depth.

21. Catheter attachment according to claim 18 or 19, characterized in that the line of weakened material consists of a row of indentations or perforations.

22. Catheter attachment according to one of claims 13 to 21, characterized in that the carrier portion (26) is produced from plastic integrally with the attachment (1) and is arranged on the underside of the attachment (1) running tangentially thereto.

23. Catheter attachment according to one of claims 13 to 22, characterized in that the corners of the plate member are rounded and that the two plates (4, 5, 27) and/or the carrier portion (26) have groove profiles (20; 24; 29) on at least one face.

## Revendications

1. Embout pour cathéter destiné à être utilisé dans des appareils de ponction de veines, dans lequel un tuyau de cathéter (3) ou une canule métallique est fixé sur une pièce d'embout creuse (1), laquelle porte deux plaques sensiblement planes (4, 5; 27) reliées l'une à l'autre, qui, lorsque la pièce d'embout (1) est fixée sur la peau du patient reposent sur la peau en s'étendant selon deux côtés opposés et qui sont pivotables vers le haut à partir de cette position, caractérisé en ce que les deux plaques (4, 5; 27) sont reliées rigidement l'une à l'autre et sont montées sur l'embout (1) par un axe (6; 25) s'étendant perpendiculairement à l'axe longitudinal de l'embout de cathéter, et qu'est prévue une butée sur laquelle viennent s'appuyer les plaques (4, 5; 27) dans leur position basculée vers le haut sur la pièce d'embout (1).

2. Embout pour cathéter selon la revendication 1, caractérisé en ce que la butée est constituée par un étrier (7; 28) qui relie les deux plaques (4, 5; 27) l'une à l'autre et dont l'extrémité tournée vers l'axe (6; 25) s'applique contre la pièce d'embout (1) lorsque les plaques (4, 5; 27) sont basculées en position haute.

3. Embout pour cathéter selon la revendication 2, caractérisé en ce que l'étrier (7; 28) a une forme bombée conique et en coupe une section semi-circulaire, et en ce que le diamètre de son extrémité tournée vers l'axe (6, 35) est le plus grand.

4. Embout de cathéter selon la revendication 2 ou 3, caractérisé en ce que l'étrier (7; 28) s'applique lorsque les plaques (4, 5; 27) sont en contact avec la peau du patient, sur une saillie axiale (boisseau de protection 2) de la pièce d'embout (1) et maintient en l'air la pièce d'embout (1) obliquement vers l'arrière.

5. Embout de cathéter selon la revendication 4, caractérisé en ce que l'angle entre l'axe longitudinal de la pièce d'embout (1) et les plaques couchées (4, 5; 27) est de 5° environ 20°.

6. Embout pour cathéter selon l'une des revendications 1 à 5, caractérisé en ce que les plaques (4, 5) peuvent être verrouillées dans les deux positions au moyen de cames d'arrêt (12) prévues sur la pièce d'embout (1).

7. Embout pour cathéter selon l'une des revendications 1 à 6, caractérisé en ce que l'axe est conformé en axe de pivotement (6) et, en position haute des plaques (4, 5) il est monté sur leur bord inférieur en s'étendant tangentiellement au côté inférieur de la pièce d'embout (1).

8. Embout pour cathéter selon la revendication 7, caractérisé en ce que l'axe de pivotement (6) est constitué de tourillons latéraux (19; 22) prévus sur la pièce d'embout (1) ou sur les deux plaques (4, 5) et qui s'engagent dans des ouvertures-paliers latérales (18, 23) prévues sur les deux plaques (4, 5) ou dans la pièce d'embout (1).

9. Embout pour cathéter selon la revendication 8, caractérisé en ce que les tourillons (19; 22) ou les ouvertures-paliers (18; 23) de chacune des plaques (4, 5) sont formés dans des pattes (17; 21) sensiblement perpendiculaires aux plaques (4, 5).

10. Embout pour cathéter selon la revendication 8 ou 9, caractérisé en ce que les ouvertures-paliers (18; 23) dans les pattes (17) ou dans la pièce d'embout (1) sont conformés en forme de griffes ouvertes sur un côté et peuvent être montés par encliquetage élastique sur les tourillons (19; 22) prévus sur la pièce d'embout (1) ou les pattes (21).

11. Embout pour cathéter selon la revendication 7, caractérisé en ce que l'axe de pivotement (6) a la forme d'une pièce unitaire (6a, 6b) qui est montée dans une ouverture-palier (16) de la pièce d'embout (1).

12. Embout pour cathéter selon la revendication 11, caractérisé en ce que l'axe de pivotement (6a, 6b) est conformé pour pouvoir être cintré de façon élastique.

13. Embout pour cathéter selon la revendication 1, caractérisé en ce que l'axe est une ligne matérielle de moindre résistance (25), qui partage un corps de plaque en matière souple en une partie support (26) reliée à la pièce d'embout (1) et aux plaques pivotables (27).

14. Embout pour cathéter selon la revendication 13, caractérisé en ce que le corps de plaque est plan et que, vues en plan, les plaques (27) et la partie support (26) se présentent chacune sous une forme sensiblement rectangulaire.

15. Embout de cathéter selon la revendication 13, caractérisé en ce que le corps de plaque est plan et que, vue en plan, la partie support est triangulaire ou trapézoïdale et les plaques sensiblement rectangulaires.

16. Embout de cathéter selon la revendication 13, caractérisé en ce que la partie support (26) est plus courte, dans la direction longitudinale de la pièce d'embout (1), que les plaques (27).

17. Embout pour cathéter selon la revendication 13, caractérisé en ce que la ligne matérielle de moindre résistance (25) se trouve dans la face inférieure du corps de plaque.

18. Embout pour cathéter selon la revendication 17, caractérisé en ce que la ligne matérielle de moindre résistance (25) traverse chaque plaque (27) de l'une à l'autre extrémité.

19. Embout pour cathéter selon la revendication 17, caractérisé en ce que la ligne matérielle de moindre résistance est plus courte que chaque plaque (27).

20. Embout pour cathéter selon la revendication 18 ou 19, caractérisé en ce que la ligne matérielle de moindre résistance (25) a la forme d'une rainure rectiligne de profondeur uniforme.

21. Embout pour cathéter selon la revendication 18 ou 19, caractérisé en ce que la ligne matérielle de moindre résistance est constituée de dépressions ou de perforations disposés côte-à-côte.

22. Embout pour cathéter selon l'une des revendications 13 à 21, caractérisé en ce que la partie support (26) est fabriquée en plastique d'un seul tenant avec la pièce d'embout (1), et s'étend tangentiellement à cette dernière sur la face inférieure de la pièce d'embout (1).

23. Embout pour cathéter selon l'une des revendications 13 à 22, caractérisé en ce que les angles du corps de plaque sont arrondis, et en ce que les deux plaques (4, 5; 27) et/ou la partie support (26) présentent au moins sur une face des rainures profilées (20; 24; 29).

FIG.1

FIG.2

0 023 580

0 023 580

FIG.3

FIG.4

FIG.5

FIG.6

FIG.7

15

# FIG.8

# FIG.9

# FIG.10

# FIG.11

# FIG.12

# FIG.13

FIG.14

FIG.15

FIG.16

FIG.17

FIG.18

FIG.19

## FIG.20

**FIG.21**

**FIG.22**

**FIG.23**

FIG.24

0 023 580